Europäisches Patentamt

European Patent Office

Office européen des brevets

(19)

(11) Veröffentlichungsnummer: **0 502 321 A1**

# (12) EUROPÄISCHE PATENTANMELDUNG

(21) Anmeldenummer: **92101666.3**

(22) Anmeldetag: **01.02.92**

(51) Int. Cl.5: **A61M 39/00**

(30) Priorität: **06.03.91 DE 4107044**

(43) Veröffentlichungstag der Anmeldung:
**09.09.92 Patentblatt 92/37**

(84) Benannte Vertragsstaaten:
**AT DE DK ES FR GB IT SE**

(71) Anmelder: **JOKA KATHETERTECHNIK GMBH**
**Ermelesstrasse 37**
**W-7450 Hechingen(DE)**

(72) Erfinder: **Sunnanväder, Lars,**

**Dipl.-Betriebswirt**
**Silberburgstrasse 6**
**W-7450 Hechingen(DE)**
Erfinder: **Hartig, Tomas**
**Läviweg 22**
**W-7450 Hechingen(DE)**

(74) Vertreter: **Weber, Dieter, Dr. et al**
**Dr. Dieter Weber, Dipl.-Phys. Klaus Seiffert,**
**Dr. Winfried Lieke, Patentanwälte,**
**Gustav-Freytag-Strasse 25, Postfach 6145**
**W-6200 Wiesbaden 1(DE)**

(54) **Absperrvorrichtung an einer Flüssigkeitsleitung, insbesondere einer Flüssigkeitsentnahme- oder Infusionseinrichtung.**

(57) Die vorliegende Erfindung betrifft eine Absperrvorrichtung an einer Flüssigkeitsleitung, insbesondere einer Flüssigkeitsentnahme- oder Infusionseinrichtung, mit einem büchsenförmigen Gehäuse (2) mit einer seitlichen Öffnung (4) zwischen seinen Enden, in deren Bereich ein an der Innenwand des Gehäuses (2) dichtend anliegendes, elastisches Schlauchstück (7) eingesetzt ist, mit einer im Bereich der Öffnung (4) seitlich am Gehäuse (2) angeordneten Führung (8) für einen Druckstift (15), der im wesentlichen zwischen zwei Stellungen radial zum Gehäuse (2) durch die seitliche Öffnung (4) bewegbar und in mindestens einer dieser Stellungen fixierbar ist, in welcher er das elastische Schlauchstück (7) zusammendrückt. Um die Handhabung einer solchen Vorrichtung dahingehend zu verbessern, daß sowohl das Schließen als auch das Öffnen des Ventils allein mit einer Hand leicht bewerkstelligt werden kann, ohne daß heftige, ruckartige Bewegungen an dem Gehäuse auftreten, wird erfindungsgemäß vorgeschlagen, daß mindestens eine sich im wesentlichen parallel zum Druckstift (15) erstreckende und mit dem Stift (15) verbundene federnde Zunge (3) vorgesehen ist, welche ein Riegelelement (6) aufweist, daß in der fixierbaren Stellung mit einem passenden Gegenstück (9), das fest mit der Führung (8) und/oder dem Gehäuse (2) verbunden ist, durch die Federspannung der Zunge (3) in Eingriff bringbar ist und durch einen Druck gegen die Federkraft der Zunge (3) außer Eingriff bringbar ist.

Fig. 2
(A-A)

EP 0 502 321 A1

Die vorliegende Erfindung betrifft eine Absperrvorrichtung an einer Flüssigkeitsleitung, insbesondere einer Flüssigkeitsentnahme-oder Infusionseinrichtung, mit einem büchsenförmigen Gehäuse mit einer seitlichen Öffnung zwischen seinen Enden, in deren Bereich ein an der Innenwand des Gehäuses dichtend anliegendes, elastisches Schlauchstück eingesetzt ist, mit einer im Bereich der Öffnung seitlich am Gehäuse angeordneten Führung für einen Druckstift, der im wesentlichen zwischen zwei Stellungen radial zum Gehäuse durch die seitliche Öffnung bewegbar und mindestens in einer dieser Stellungen fixierbar ist, in welcher er das elastische Schlauchstück zusammendrückt.

Eine solche Absperrvorrichtung bzw. ein solches Absperrventil, das beispielsweise in der Medizin als Katheterverschluß dient, ist aus der deutschen Patentanmeldung P 37 31 242.1 bekannt. Bei der bekannten Vorrichtung wird die Fixierung des Absperrventils in der geschlossenen Stellung, d.h. in der Stellung in welcher der Stift das elastische Schlauchstück so zusammenquetscht, daß der Durchfluß durch dieses Schlauchstück unterbrochen wird, dadurch erreicht, daß der Druckstift im Innern einer Kappe angebracht ist, welche eine Führungshülse umgreift und mit einem elastischen Mantel und einem Schnappring versehen ist. Der Schnappring hintergreift einen an der Führungshülse angebrachten Wulst oder Vorsprung.

Diese bekannte Vorrichtung erleichtert bereits die Handhabung und insbesondere das Schließen derartiger Absperrventile allein mit einer Hand. Außerdem sind solche Absperrventile relativ einfach und preiswert herstellbar. Etwas schwieriger ist jedoch das Öffnen eines solchen Absperrventils, da zum Lösen der Kappe, d.h. um den Schnappring über den an der Führungshülse gebildeten Vorsprung hinwegzuziehen, eine Zugkraftan der Kappe einerseits und an dem Gehäuse bzw. der damit fest verbundenen Führungshülse andererseits ausgeübtwerden muß, was mit einer Hand nur schwer möglich ist. Die Erfindung hat sich daher zum Ziel gesetzt, die bekannte Vorrichtung dahingehend weiterzuverbessern, daß sowohl das Schließen als auch das Öffnen des Ventils allein mit einer Hand leicht bewerkstelligt werden kann, ohne daß heftige, ruckartige Bewegungen an dem Gehäuse auftreten, die leicht zu Verletzungen des Patienten oder zu Leckagen des Systems führen können. Außerdem sollen die Vorteile der einfachen und preiswerten Herstellbarkeit nach wie vor gewahrt bleiben.

Diese Aufgabe wird dadurch gelöst, daß mindestens eine, sich im wesentlichen parallel zu dem Druckstift erstreckende und mit dem Druckstift verbundene, federnde Zunge vorgesehen ist, welche ein Riegelelement aufweist, das in der fixierbaren Stellung der Vorrichtung mit einem passenden Gegenstück, welches fest mit der Führung und/oder dem Gehäuse verbunden ist, durch die Federspannung der Zunge in Eingriff bringbar und durch einen Druck gegen die Federkratt der Zunge außer Eingriff bringbar ist. Diese Gestaltung der Absperrvorrichtung bzw. des Ventils hat den Vorteil, daß das Verschließen des Ventils nach wie vor in sehr einfacher Weise mit einer Hand erfolgen kann, indem nämlich beispielsweise zwei Finger auf der dem Druckstift gegenüberliegenden Seite des Gehäuses hinter dieses gelegt und mit dem Daumen auf den Druckstift in Richtung des Gehäuses eine Kraft ausgeübt wird. Sofern das Gehäuse gegenüber dem Druckstift auf einer hinreichend festen Unterlage aufliegt, genügt sogar ein einfacher Druck mit einem Daumen oder Finger auf den Druckstift in Richtung des Gehäuses zum Verschließen der Absperrvorrichtung. Die Zunge erstreckt sich im wesentlichen parallel zum Druckstift und wird mit diesem in Richtung auf das Gehäuse bewegt, wobei in der Stellung, in welcher der Druckstift das elastische Schlauchstück vollständig zusammenquetscht und somit verschließt, die Zunge unter der Wirkung ihrer Federkraft in eine verriegelte Stellung einschnappt, wobei ihr Riegelelement ein entsprechendes Gegenstück hintergreift, das mit dem Gehäuse bzw. der Führung für den Druckstift fest verbunden ist. Zum Lösen dieser Verriegefung braucht lediglich ein seitlicher Druck auf die federnde Zunge in Richtung der entriegelten Stellung ausgeübt zu werden, so daß das Riegelelement außer Eingriff kommt, wobei das elastisch zusammengedrückte Schlauchstück selbsttätig den Druckstift in radialer Richtung wegbewegt, der dabei auch die federnde Zunge mitnimmt. Beide Vorgänge können sehr leicht mit den Fingern einer Hand ausgeführt werden, ohne daß größere Erschütterungen auf das Gehäuse und die damit verbundenen Teile übertragen werden.

Besonders zweckmäßig ist eine Ausführungsform der Erfindung, bei welcher zwei federnde Zungen auf gegenüberliegenden Seiten des Druckstiftes angeordnet sind, die eine von dem Druckstift weggerichtete Federspannung aufweisen. Zum Lösen einer solchen Vorrichtung brauchen lediglich die beiden gegenüberliegenden federnden Zungen zwischen Daumen und Zeigefinger ergriffen und zusammengedrückt zu werden, woraufhin die Entriegelung und Öffnung des Absperrventils unmittelbar und ohne weitere Kraftausübung erfolgt.

Zweckmäßigerweise hat das Riegelelement die Form eines nasenförmigen Vorsprunges, welcher einen entsprechenden Gegenvorsprung bzw. einen Rand oder eine Kante des Gegenstückes hintergreift.

Bevorzugt ist eine Ausführungsform der Erfindung, bei welcher das Gegenstück für das Riegelelement an einer die Führung für den Druckstift im Abstand umgebenden Hülse angeordnet ist, welche mindestens teilweise auch die federnde Zunge(n) umschließt, die dementsprechend mit federnder Vorspannung an der Innenwand der Hülse anliegt (anliegen). Bei einer von dem Druckstift weggerichteten Vorspannung der federnden Zunge versteht es sich von selbst, daß die Gegenstücke zum Riegelelement in dieser Richtung noch weiter vom Druckstift entfernt sein müssen, was man in einfacher Weise mit der genannten Hülse erreicht. Dabei kann das Gegenstück, mit welchem das Riegelelement der federnden Zunge in Eingriff treten soll, durch den Rand einer Aussparung der Hülse oder aber durch den unteren Rand der Hülse selbst gebildet werden. Besonders bevorzugt ist eine Ausführungsform der Erfindung, bei welcher die Hülse an gegenüberliegenden Seiten je eine Aussparung aufweist, durch welche mindestens in der fixierten Stellung des Druckstiftes je ein nockenartiger Vorsprung der beiden Federzungen hindurch und über die Umfangsoberfläche der Hülse hinausragt. Diese nockenartigen Vorsprünge können sehr leicht mit den Fingern einer Hand erfaßt und zusammengedrückt werden, wodurch die Riegelelement der federnden Zungen außer Eingriff mit ihren Gegenstücken gebracht werden.

Dies kann sogar allein durch Ertasten der nokkenartigen Vorsprünge geschehen, da diese mindestens in der verriegelten Stellung über die Umfangsoberfläche der Hülse hinausragen. Andererseits sind die federnden Zungen durch die umgebende Hülse weitgehend gegen unbeabsichtigtes Lösen geschützt, zumal zum Öffnen des Ventils ein gleichzeitiger Druck auf beide federnden Zungen von gegenüberliegenden Seiten her erforderlich ist.

Zweckmäßigerweise weist der Druckstift an seiner Oberseite eine Platte auf, die über den Durchmesser des Stiftes hinausragt und von der sich auf gegenüberliegenden Seiten des Druckstiftes die beiden Zungen im wesentlichen parallel zum Druckstift erstrecken.

Die Führung für den Druckstift wird am besten durch eine innere Führungshülse gebildet, die fest und gegebenenfalls auch einstückig mit dem Gehäuse verbunden sein kann und konzentrisch zu der seitlichen Öffnung im Gehäuse liegt.

Weitere Vorteile, Merkmale und Anwendungsmöglichkeiten der vorliegenden Erfindung werden deutlich anhand der folgenden Beschreibung einer bevorzugten Ausführungsform und der dazugehörigen Figuren. Es zeigen:

Figur 1    eine Seitenansicht des Absperrventils,

Figur 2    einen Schnitt entlang der Ebene A-A in Figur 1,

Figur 3    eine Ansicht auf die Absperrvorrichtung nach Figur 1 von oben,

Figur 4    eine Ansichtvon links auf das in Figur 3 dargestellte Absperrventil,

Figur 5    eine andere Ausführungsform der Erfindung im Längsschnitt,

Figur 6    einen Horizontalschnitt entlang der Linie C-C in Figur 5,

Figur 7    einen Längsschnitt durch das Ventil nach Figur 5 im geschlossenen Zustand und

Figur 8    einen Querschnitt entlang der Linie B-B in Figur 7.

Die beiden in den Figuren dargestellten Ausführungsformen sind weitgehend identisch und weisen für die identischen Teile übereinstimmende Bezugszahlen auf.

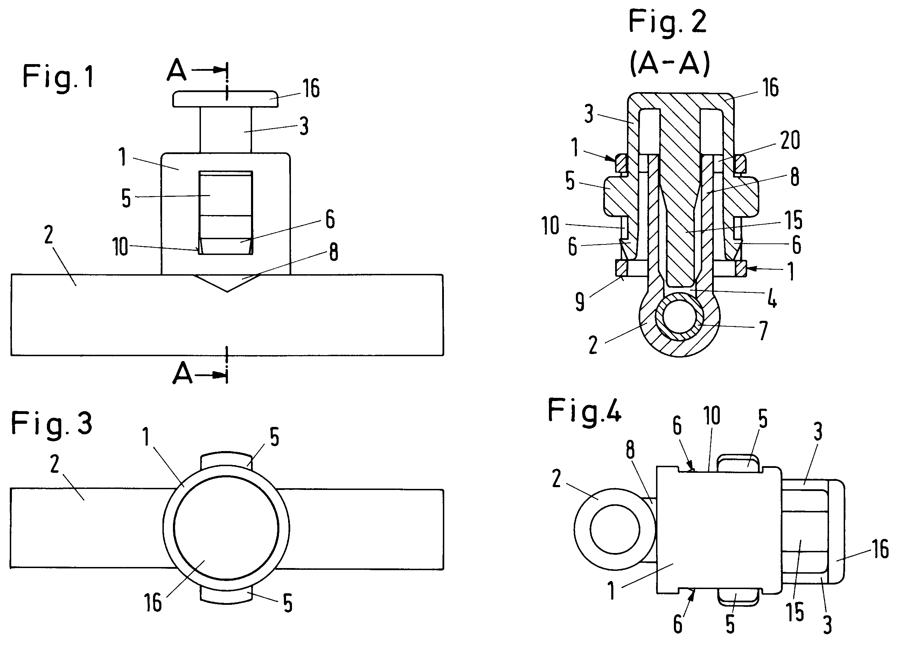

In Figur 1 erkennt man ein rohrförmiges Gehäuse 2, mit einer seitlich angesetzten Führungshülse 8, die konzentrisch eine nur in Figur 2 erkennbare Öffnung 4 umgibt. Wie man in Figur 2 weiterhin deutlich sieht, ist in das büchsenförmige Gehäuse 2 ein elastisches Schlauchstück 7 eingesetzt, das dicht an der Innenwand des Gehäuses 2 anliegt und sich über den Bereich der seitlichen Öffnung 4 hinweg erstreckt. Die Länge des Schlauchstückes 7 ist dabei so bemessen, daß seine Enden auf jeden Fall über ihren gesamten Umfang auch dann dichtend an der Innenwand des Gehäuses 2 anliegen, wenn im mittleren Bereich das elastische Schlauchstück 7 durch den Druckstift 15 zusammengequetscht ist.

Die jeweils fest zusammenhängenden Teile sind in Figur 2 jeweils durch gleiche Schraffuren dargestellt. Man erkennt daran, daß das Gehäuse 2 mit der zylindrischen inneren Führungshülse 8 und einer äußeren Hülse 1 fest verbunden ist. Der Druckstift 15 ist zusammen mit einer Kopfplatte 16 und den federnden Zungen 3, die auf gegenüberliegenden Seiten sich parallel zu dem Druckstift von der Kopfplatte 16 aus erstrecken, einstückig hergestellt. Die Hülse 1 weist zwei gegenüberliegende, im wesentlichen rechteckige Aussparungen 10 auf, durch welche zwei nockenartige Vorsprünge 5 hindurchragen, welche auf den außen liegenden Seitenflächen der federnden Zungen 3 angebracht sind.

Während also die oberen Enden der federnden Zungen 3 mit dem Druckstift 15 verbunden sind, werden die unteren Enden der federnden Zungen 3 von nasenförmigen Riegelelementen 6 gebildet, welche den unteren Rand 9 der Hülse 1 hintergreifen, wenn der Druckstift 15 in das Gehäuse 2 hineingedrückt wird und das elastische Schlauchstück 7 zusammenquetscht. Die hervorstehenden Nocken 5 sind auch in den Figuren 3 und 4 deutlich erkennbar. Die federnden Zungen liegen mit einer gewissen, vom Druckstift 15 weggerichteten Vorspannung an der Innenwand der Hülse 1 an, und zwar sowohl im oberen Bereich, d.h. oberhalb der Aussparung 10, als auch im unteren Bereich mit dem Enden der Federzungen. Aufgrund der Nasenform der Riegelelemente 6 gleitet die Schrägfläche am unteren äußeren Rand der federnden Zungen 3 leicht über die obere Innenkante des unteren Randes der Aussparung 10 hinweg, wobei die federnden Zungen weiter unter Vorspannung gesetzt werden, bis die Riegelelemente 6 unter dem unteren Rand (9) der Hülse 1 verrasten können und diesen unteren Rand hintergreifen.

Die nockenförmigen Vorsprünge 5 verschieben sich dabei in der rechteckig länglichen Aussparung 10 nach unten bzw. in Richtung des Gehäuses 2, liegen aber nach wie vor im Bereich dieser Aussparung und ragen über die äußere Umfangsoberfläche der Hülse 1 hinaus, wie sich aus den in den Figuren dargestellten Abmessungen der Nocken 5, der Zungen 3 mit ihren Riegelelementen 6 und der Hülse 1 mit der Aussparung 10 ohne weiteres ergibt. Dabei sind die Aussparungen 10 und die Federzungen so ausgerichtet, daß sie quer zur Längserstreckung des Gehäuses 2 auf gegenüberliegenden Seiten der Hülse 1 angeordnet sind, so daß die Handhabung hierdurch zusätzlich erleichtert wird, da die Finger nicht auf das Gehäuse 2 stoßen, wenn die nockenartigen Vorsprünge ergriffen und zusammengedrückt werden. Das Zusammendrücken der beiden nockenartigen Vorsprünge 5 zwischen den Fingern einer Hand bringt, in der fixierten Stellung des Druckstiftes, die nasenförmigen Riegelelemente 5 außer Eingriff mit dem unteren Rand 9 der Hülse 1, so daß der Druckstift mit den federnden Zungen 3 durch die elastische Rückstellkraft des Schlauchstückes 7 nach oben gedrückt wird und in etwa wieder die in den Figuren dargestellte Stellung einnimmt.

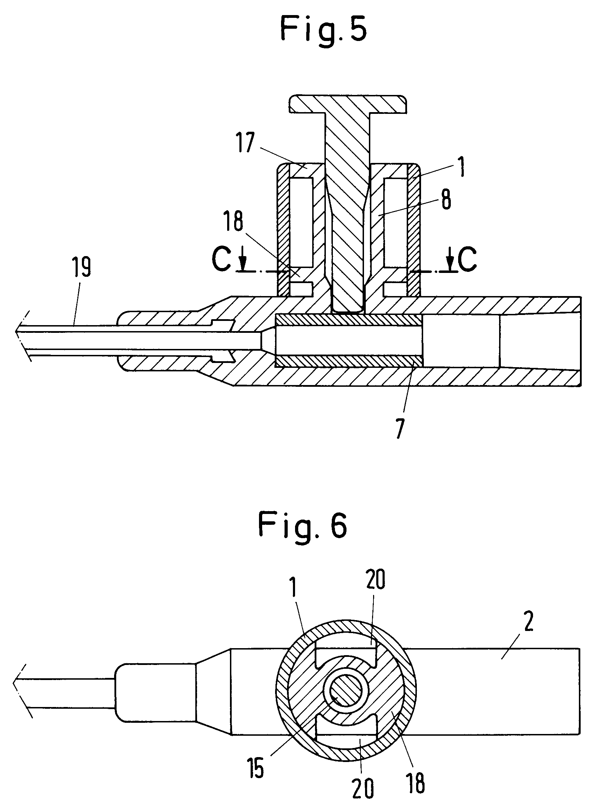

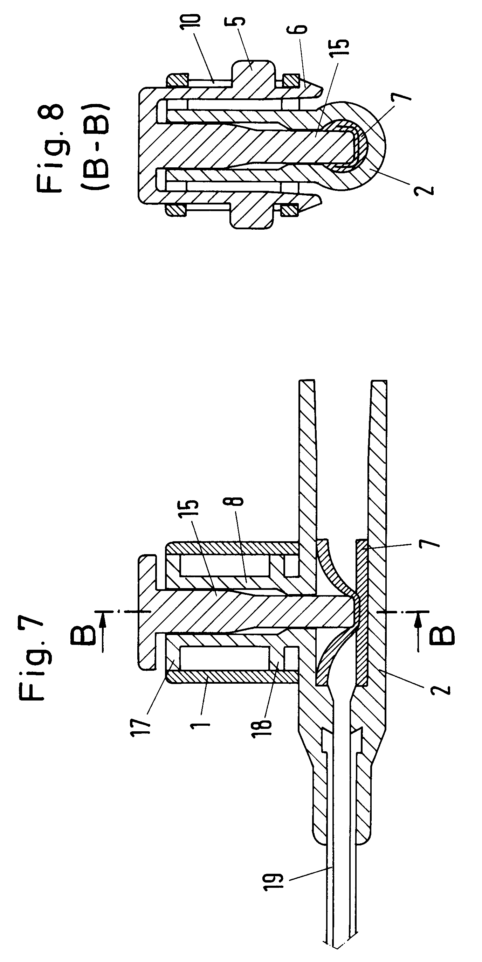

Eine weitere Ausführungsform der Erfindung ist in den Figuren 5 bis 8 dargestellt, die sich von der Ausführungsform nach den Figuren 1 bis 4 im wesentlichen nur dadurch unterscheidet, daß sie eine an einem Ende des Gehäuses 2 angesetzte Kanüle 19 und ein passendes Mundstück des Gehäuses 2 aufweist. In allen anderen Merkmalen stimmt diese Ausführungsform mit der in den Figuren 1 bis 4 dargestellten Ausführungsform überein. Dargestellt sind in den Figuren 5 bis 8 jedoch zusätzliche Schnittebenen und insbesondere auch der geschlossene Zustand des Ventils in den Figuren 7 und 8. Im Längsschnitt der Figuren 5 und 7 erkennt man zwei Flansche 17 und 18, die mit der inneren Hülse 8 einstückig sind und im fertig montierten Zustand eine feste Verbindung mit der Hülse 1, z.B. durch Schweißen oder Kleben, aufweisen. Die genaue Form des Flansches 18 ist im Horizontalschnitt der Figur 6 zu erkennen. Man erkennt dabei insbesondere auch den zwischen Flansch 18 und Hülse 1 verbleibenden Freiraum 20 für die in der Hülse 1 geführten federnden Zungen 3. Der Flansch 17 hat im wesentlichen die gleiche Form wie der Flansch 18. Aus Figur 6 ergibt sich, daß in der in Figur 2 dargestellten Schnittebene A-A die Flansche 17, 18 nicht erkennbar sind.

Figur 7 stellt im wesentlichen denselben Längsschnitt durch die zweite Ausführungsform dar, wie Figur 5, jedoch mit herabgedrücktem Druckstift 15, so daß man sieht, wie der Druckstift 15 das elastische Schlauchstück 7 zusammenquetscht und verschließt. Dies wird auch im Querschnittbild der Figur 8 deutlich, die außerdem zeigt, wie die Riegelelemente 6 unter dem unteren Rand der Hülse 1 verrasten.

Die gesamte Vorrichtung besteht aus` nur wenigen Teilen, nämlich dem Gehäuse 2 mit der einstückig angesetzten Führungshülse 8, die wiederum über Flansche 17, 18 mit der Außenhülse 1 verbunden ist. Es versteht sich, daß allein die Verbindung über den Flansch 17 ausreichen würde und der Flansch 18 der Außenhülse 1 nur noch zusätzliche Festigkeit verleiht. Die weiteren Teile sind der Druckstift 15 mit der einstückig angeformten Platte 16 und den Federzungen 3 sowie das eingesetzte elastische Schlauchstück 7.

Die Vorrichtung ist in ihrer Handhabung absolut sicher und einfach. Die Vorrichtung läßt sich in sehr kleinen Abmessungen und mit einem äußerst geringen Gewicht herstellen und bleibt dennoch leicht handhabbar. Diese Vorteile sind im medizinischen Bereich von großer Bedeutung.

**Patentansprüche**

1. Absperrvorrichtung an einer Flüssigkeitsleitung, insbesondere einer Flüssigkeitsentnahme- oder Infusionseinrichtung, mit einem büchsenförmigen Gehäuse (2) mit einer seitlichen Öffnung (4) zwischen seinen Enden, in deren Bereich ein an der Innenwand des Gehäuses (2) dichtend anliegendes, elastisches Schlauchstück (7) eingesetzt ist, mit einer im Bereich der Öffnung (4) seitlich am Gehäuse (2) angeordneten Führung (8) für einen Druckstift (15), der im wesentlichen zwischen zwei Stellungen radial zum Gehäuse (2) durch die seitliche Öffnung (4) bewegbar und in mindestens einer dieser Stellungen fixierbar ist, in welcher er das elastische Schlauchstück (7) zusammendrückt, dadurch gekennzeichnet, daß mindestens eine sich im wesentlichen parallel zum Druckstift (15) erstreckende und mit dem Druckstift (15) verbundene federnde Zunge (3) vorgesehen ist, welche ein Riegelelement (6) aufweist, das in der fixierbaren Stellung mit einem passenden Gegenstück (9), das fest mit der Führung (8) und/oder dem Gehäuse (2) verbunden ist, durch die Federspannung der Zunge (3) in Eingriff bringbar ist und durch einen Druck gegen die Federkraft der Zunge (3) außer Eingriff bringbar ist.

**2.** Vorrichtung nach Anspruch 1, dadurch gekennzeichnet, daß zwei federnde Zungen (3) auf gegenüberliegenden Seiten des Druckstiftes (15) angeordnet sind, welche eine vom Druckstift (15) weggerichtete Vorspannung aufweisen.

**3.** Vorrichtung nach Anspruch 1 oder 2, dadurch gekennzeichnet, daß das Riegelelement (6) ein nasenförmiger Vorsprung ist.

**4.** Vorrichtung nach einem der Ansprüche 1 bis 3, dadurch gekennzeichnet, daß das Gegenstück für das Riegelelement (6) an einer die Druckstiftführung (8) im Abstand umgebenden Hülse (1) angeordnet ist, welche mindestens teilweise auch die federnde Zunge (3) einschließt, die mit federnder Vorspannung an der Innenwand der Hülse (1) anliegt.

**5.** Vorrichtung nach Anspruch 4, dadurch gekennzeichnet, daß das Gegenstück für das Riegelelement (6) durch eine Aussparung in der Hülse (1) oder durch den unteren Rand (9) der Hülse (1) gebildet wird.

**6.** Vorrichtung nach Anspruch 4 oder 5, dadurch gekennzeichnet, daß die Hülse (1) an gegenüberliegenden Seiten je eine Aussparung (10) aufweist, durch welche in der fixierten Stellung des Druckstiftes (15) je ein nockenartiger Vorsprung (5) über die Umfangsoberfläche der Hülse (1) hinausragt, wobei die nockenartigen Vorsprünge (5) an je einer federnden Zunge (3) angeordnet sind.

**7.** Vorrichtung nach einem der Ansprüche 1 bis 6, dadurch gekennzeichnet, daß an der Oberseite des Druckstiftes (15) eine den Durchmesser des Stiftes (15) überschreitende Platte (16) angeordnet ist, von der sich auf gegenüberliegenden Seiten des Druckstiftes (15) zwei federnde Zungen (3) parallel zum Stift (15) erstrecken.

**8.** Vorrichtung nach einem der Ansprüche 1 bis 7, dadurch gekennzeichnet, daß die Stiftführung (8) eine zur äußeren Hülse (1) konzentrische und mit der äußeren Hülse (1) und dem Gehäuse (2) fest verbundene innere Hülse (8) ist.

Fig.1

A

16

3

1

5

6

10

8

2

A

Fig. 2
(A-A)

16

3

1

5

10

6

9

2

20

8

15

6

1

4

7

Fig. 3

1

5

2

16

5

Fig.4

6   10   5

8

2

3

1

16

6   5

15   3

EP 0 502 321 A1

6

# Fig.5

# Fig. 6

Fig. 8 (B-B)

Fig. 7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | WO-A-9 100 970 (MEDINORM AKTIENGESELLSCHAFT MEDIZINTECHNISCHE PRODUKTE) <br> * Seite 9, Absatz 3 –Absatz 4; Abbildungen 3-5 * <br> --- | 1,7 | A61M39/00 |
| D,A | DE-A-3 731 242 (JOKA KATHETERTECHNIK GMBH) <br> * Anspruch 1; Abbildungen 1-5 * <br><br> ----- | 1,7 | |
| | | | **RECHERCHIERTE SACHGEBIETE (Int. Cl.5 )** <br><br> A61M <br> F16K |

Der vorliegende Recherchenbericht wurde für alle Patentansprüche erstellt

| Recherchenort | Abschlußdatum der Recherche | Prüfer |
|---|---|---|
| BERLIN | 20 MAI 1992 | ROLAND A. |

EPO FORM 1503 03.82 (P0403)